(19) Europäisches Patentamt
European Patent Office
Office européen des brevets

(11) EP 2 287 230 B1

(12) **EUROPEAN PATENT SPECIFICATION**

(45) Date of publication and mention of the grant of the patent:
**26.09.2012 Bulletin 2012/39**

(21) Application number: **09750688.5**

(22) Date of filing: **20.05.2009**

(51) Int Cl.:
***C08G 69/40*** *(2006.01)* ***A61K 31/337*** *(2006.01)*
***A61K 47/34*** *(2006.01)* ***A61K 47/48*** *(2006.01)*
***A61P 35/00*** *(2006.01)* ***C08G 81/00*** *(2006.01)*
*A61K 9/00 (2006.01)* *C08G 69/10 (2006.01)*

(86) International application number:
**PCT/JP2009/059633**

(87) International publication number:
**WO 2009/142326 (26.11.2009 Gazette 2009/48)**

(54) **Docetaxel polymer derivative, method for producing same and use of same**

Docetaxelpolymerderivat, Verfahren zu seiner Herstellung und Verwendung

Dérivé de polymère de docétaxel, procédé pour sa préparation et son utilisation

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **23.05.2008 JP 2008135933**
**12.03.2009 JP 2009060129**

(43) Date of publication of application:
**23.02.2011 Bulletin 2011/08**

(73) Proprietor: **NanoCarrier Co., Ltd.**
**Kashiwa-shi, Chiba 277-0882 (JP)**

(72) Inventors:
- **HARADA, Mitsunori**
  **Kashiwa-shi**
  **Chiba 277-0882 (JP)**
- **SAITO, Hiroyuki**
  **Kashiwa-shi**
  **Chiba 277-0882 (JP)**
- **KATO, Yasuki**
  **Kashiwa-shi**
  **Chiba 277-0882 (JP)**

(74) Representative: **Dean, John Paul et al**
**Withers & Rogers LLP**
**4 More London Riverside**
**London**
**SE1 2AU (GB)**

(56) References cited:
**EP-A1- 1 415 648** **EP-A1- 1 508 331**
**WO-A1-03/000771** **WO-A1-2007/111211**
**WO-A2-2005/079861** **JP-A- 8 310 970**
**US-A1- 2004 056 372** **US-A1- 2010 234 537**

- **SUNG BUM LA ET AL: "PREPARATION AND CHARACTERIZATION OF THE MICELLE-FORMING POLYMERIC DRUG INDOMETHACIN-INCORPORATED POLY(ETHYLENE OXIDE)-POLY(BETA- BENZYL L-ASPARTATE) BLOCK COPOLYMER MICELLES", JOURNAL OF PHARMACEUTICAL SCIENCES, AMERICAN PHARMACEUTICAL ASSOCIATION, WASHINGTON, US, vol. 85, no. 1, 1 January 1996 (1996-01-01), pages 85-90, XP000543852, ISSN: 0022-3549, DOI: 10.1021/JS950204R**
- **PROMPRUK K ET AL: "Synthesis of a novel PEG-block-poly(aspartic acid-stat-phenylalanine) copolymer shows potential for formation of a micellar drug carrier", INTERNATIONAL JOURNAL OF PHARMACEUTICS, ELSEVIER BV, NL, vol. 297, no. 1-2, 13 June 2005 (2005-06-13), pages 242-253, XP004904008, ISSN: 0378-5173**
- **MASAYUKI YOKOYAMA ET AL: "POLYMER MICELLES AS NOVEL DRUG CARRIER: ADRIAMYCIN-CONJUGATED POLY(ETHYLENE GLYCOL)-POLY(ASPARTIC ACID) BLOCK COPOLYMER", JOURNAL OF CONTROLLED RELEASE, ELSEVIER, AMSTERDAM, NL, vol. 11, no. 1 - 03, 1 January 1990 (1990-01-01), pages 269-278, XP000113384, ISSN: 0168-3659, DOI: 10.1016/0168-3659(90)90139-K**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 287 230 B1

## Description

### TECHNICAL FIELD

The present invention relates to a polymer derivative of docetaxel, as well as a method of preparing the derivative and uses of the derivative.

### BACKGROUND ART

Docetaxel is a taxane anti-cancer agent semisynthesized from an extract of Taxus baccata needles. It promotes tubulin polymerization to form stable microtubules and prevents its depolymerization. It also forms morphologically abnormal microtubule bundles in the cells. It is known to arrest mitosis through these effects.

In general, taxane anti-cancer agents are poorly soluble in water, and therefore require use of special organic solvents for its administration to humans. To overcome the problem of such low water-solubility, methods have been developed for improving the water-solubility of a taxane anti-cancer agent by encapsulating it with a block copolymer having a hydrophilic segment and a hydrophobic segment to form polymer micelles through hydrophobic interaction (Patent documents 1 and 2).

Patent document 3 discloses that the water-solubility of doxorubicin hydrochloride can be improved by linking it to a block copolymer including polyethylene glycol and polyaspartic acid via an amide linkage.

Patent document 4 discloses a polymer derivative of SN-38 in which the phenolic hydroxyl group of SN-38 is bound to a block copolymer having polyethylene glycol and polyglutamic acid via an ester linkage.

Patent document 5 discloses a polymer derivative of a taxane such as docetaxel, in which the alcoholic hydroxyl group of taxane is attached to a block copolymer including polyethylene glycol and polyaspartic acid.

### PRIOR ART REFERENCES

### PATENT DOCUMENTS

Patent document 1: EP1127570 A
Patent document 2: WO2004/082718
Patent document 3: JP02-300133 A
Patent document 4: WO2004/039869
Patent document 5: WO2007/111211

### DISCLOSURE OF THE INVENTION

### PROBLEMS TO BE SOLVED BY THE INVENTION

However, despite the presence of the aforementioned prior art, there is still a demand for a derivative of docetaxel with reduced side effects and improved efficacy.

In view of the above problems, the present invention was made in order to further reduce side effects of docetaxel and improve efficacy thereof.

### MEANS TO SOLVE THE PROBLEMS

The present inventors expected that a sustained release formulation, a dosage form that regulates the amount of drug release after administration, may be effective in terms of reducing side effects, since a sudden increase in the concentration of a free drug immediately after administration of an anti-cancer agent could lead to manifestation of side effects. The present inventors also considered that such a dosage form may prolong the duration of action of a taxane such as docetaxel on tumor cells and thereby enhance the efficacy thereof.

Further, after intensive and extensive efforts to develop an anti-cancer agent with reduced side effects and improved efficacy, the present inventors have found that the release rate of docetaxel from a polymer derivative of docetaxel can be controlled by adjusting the ratio and/or the number of docetaxel molecules linked to a block copolymer, and have succeeded in obtaining a novel polymer derivative of docetaxel which encapsulates a high content of docetaxel, to thereby complete the present invention.

The subject matters of the present invention are illustrated below:

(1) A method of preparing a polymer derivative of docetaxel provided by an ester linkage between at least one hydroxyl group of docetaxel and a carboxyl group in an aspartic acid side chain of a block copolymer having polyethylene glycol and polyaspartic acid. The method includes a step of adjusting the ratio and/or the number of docetaxel molecules linked to the block copolymer to thereby control the release rate of docetaxel from the resultant polymer derivative of docetaxel;

(2) The method according to (1), wherein the adjusting includes (i) adjusting the ratio of the number of linked docetaxel molecules to the total number of aspartic acid repeating units per molecule of the block copolymer to 28% or higher, and/or (ii) adjusting the number of linked docetaxel molecules per molecule of the block copolymer to 11 or more, to thereby control the release rate of docetaxel from the resultant polymer derivative of docetaxel in a 0.1 M sodium phosphate buffer, pH 7.4, at 37°C in 24 hours to 29% or lower;

(3) A polymer derivative of docetaxel provided by an ester linkage between at least one hydroxyl group of docetaxel and a carboxyl group of an aspartic acid side chain of a block copolymer having polyethylene glycol and polyaspartic acid, wherein (i) the ratio of the number of linked docetaxel molecules to the total number of aspartic acid repeating units per molecule of the block copolymer is 28% or higher, and/or (ii) the number of linked docetaxel molecules per molecule of the block copolymer is 11 or more;

(4) The polymer derivative of docetaxel according to (3), wherein the release rate of docetaxel in a 0.1 M sodium phosphate buffer, pH 7.4, at 37°C in 24 hours is 29% or lower;

(5) The polymer derivative of docetaxel according to (3) or (4), represented by formula (I):

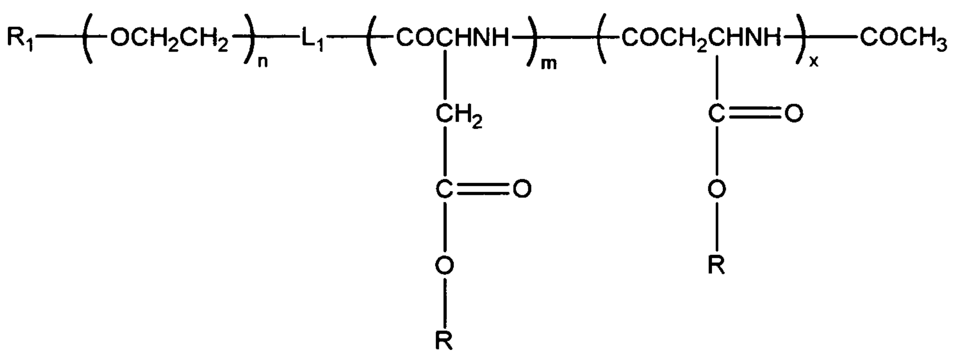

(I)

where $R_1$ is a hydrogen atom or a $C_1$-$C_6$ alkyl group, $L_1$ is a linking group, R is a hydrogen atom or a docetaxel molecule of which at least one hydroxyl group of docetaxel forms the ester linkage, n is an integer of 40 to 450, and m+x is an integer of 35 to 60, provided that 0% to 90% of m+x is x, and that when x is higher than 0, (COCHNH) units and ($COCH_2CHNH$) units are present at random; and

(6) An anti-cancer agent comprising the polymer derivative of docetaxel according to (3) to (5).

In accordance with the present invention, a polymer derivative of docetaxel having a reduced side effect and an excellent drug efficacy can be provided.

BRIEF DESCRIPTION OF THE DRAWINGS

Fig. 1 is a graph showing the results of a drug release test of polymer derivatives of docetaxel. Black circles indicate released % of docetaxel from a polymer derivative of docetaxel in which 14 molecules of docetaxel are bound per molecule of the polymer (14DTX: Example 1). White squares indicate released % of docetaxel from a polymer derivative of docetaxel in which 12 molecules of docetaxel are bound per molecule of the polymer (12DTX: Example 2). Black triangles indicate released % of docetaxel from a polymer derivative of docetaxel in which 5 molecules of docetaxel are bound per molecule of the polymer (5DTX: Comparative Example 1).

Fig. 2 is plotting the ratios of drug release 24 hours after the beginning of the experiment against the amount of docetaxel bound to the polymer, based on the results of the drug release test shown in Fig. 1. A regression line was determined from the three points.

Fig. 3 is a graph showing the results of a pharmacokinetic study in which the polymer derivative of docetaxel (PEG-pAsp-14DTX) of Example 1 or a docetaxel solution was administered to mice. Black circles indicate the concentrations of total docetaxel in the plasma when 50 mg/kg of PEG-pAsp-14DTX was administered. White squares indicate the concentrations of docetaxel released from PEG-pAsp-14DTX. Black triangles indicate the concentrations of docetaxel in the plasma when 10 mg/kg of docetaxel solved in 10% sucrose was administered. Each point represents the average of three examples and each bar represents a standard deviation.

Fig. 4 is a graph showing changes in tumor volume when the polymer derivative of docetaxel (PEG-pAsp-14DTX) of Example 1 was administered to mice bearing human prostate cancer PC-3 cells. Black circles, white triangles, white squares, and black squares indicate tumor volume changes of a group of the control (untreated), receiving DMSO solution of docetaxel, 15 mg/kg of PEG-pAsp-14DTX, and 20 mg/kg of PEG-pAsp-14DTX, respectively. Each black arrow represents a time point when the DMSO solution of docetaxel and 15 mg/kg of PEG- pAsp-14DTX were administered. Each white arrow represents a time point when 20 mg/kg of PEG-pAsp-14DTX was administered.

Fig. 5 is a graph showing changes in body weight of mice bearing human prostate cancer PC-3 cells when the polymer derivative of docetaxel (PEG-pAsp-14DTX) of Example 1 was administered to the mice. Black circles indicate changes in body weight of the control group (untreated). White triangles indicate changes in body weight of a group that a DMSO solution of docetaxel was administered. White squares indicate changes in body weight of a group that 15 mg/kg of PEG-pAsp-14DTX was administered. Black squares indicate changes in body weight of a group that 20 mg/kg of PEG-pAsp-14DTX was administered. Each black arrow represents a time point when the DMSO solution of decetaxel and 15 mg/kg of PEG-pAsp-14DTX were administered. Each white arrow represents a time point when 20 mg/kg of PEG-pAsp-14DTX was administered.

Fig. 6 is a graph showing changes in tumor volume when the polymer derivative of docetaxel (PEG-pAsp-14DTX) of Example 1 was administered to mice bearing human breast cancer MDA-MB-231 cells. Black circles indicate the control group (untreated). Black squares indicate a group that PEG-pAsp-14DTX was administered. Each arrow represents a time point when PEG-pAsp-14DTX was administered.

Fig. 7 is a graph showing changes in body weight when the polymer derivative of docetaxel (PEG-pAsp-14DTX) of Example 1 was administered to mice bearing human breast cancer MDA-MB-231 cells. Black circles indicate the control group (untreated). Black squares indicate the group that PEG-pAsp-14DTX was administered. Each arrow represents a time point when PEG-pAsp-14DTX was administered.

Fig. 8 is a graph showing changes in body weight when the polymer derivative of docetaxel (30 mg/kg of PEG-pAsp-14DTX) of Example 1, the polymer derivative of docetaxel (22 mg/kg of PEG-pAsp-5DTX) of Comparative Example 1, and a docetaxel solution (15 mg/kg) were administered to healthy mice. White circles indicate the control group (untreated). Black circles indicate the group that the PEG-pAsp-14DTX of Example 1 was administered. Black triangles indicate the group that the PEG-pAsp-5DTX of Comparative Example 1 was administered. White squares indicate the group that the docetaxel solution was administered.

DESCRIPTION OF EMBODIMENTS

The present invention provides a method of preparing a polymer derivative of docetaxel provided by an ester linkage between at least one hydroxyl group of docetaxel and a carboxyl group of an aspartic acid side chain of a block copolymer including polyethylene glycol and polyaspartic acid. The method includes a step of adjusting the ratio and/or the number of docetaxel molecules linked to the block copolymer to thereby control the release rate of docetaxel from the resultant polymer derivative of docetaxel.

The block copolymer of the present invention is not limited as long as it includes at least one polyethylene glycol and at least one polyaspartic acid. In addition to polyethylene glycol and polyaspartic acid, it may also include one or more other blocks, but even in this case, generally 90% or higher, preferably 97% or higher, and more preferably 99% or higher of the total weight of the block copolymer should be composed of polyethylene glycol and polyaspartic acid.

The method of preparing a base block copolymer, to which docetaxel is to be linked via an ester linkage, may be any method as long as it can produce a desired block copolymer. Examples of such a method include the method described in Patent document 3. Specifically, it may be obtained by reacting a starting material which is MeO-PEG-$CH_2CH_2CH_2$-$NH_2$ with N-carboxy-β-benzyl-L-aspartate (BLA-NCA) in a dehydrated organic solvent until a predetermined polymerization degree (the number of amino acid units, i.e., m+x in the equation) is achieved, and then removing benzyl groups through alkaline hydrolysis.

The term "polymer derivative of docetaxel" as used herein means a structure in which at least one of four hydroxyl groups present at positions 1, 7, 10, and 13 of 3-tert-butoxycarbonylamino-2-hydroxy-3-phenylpropionate in docetaxel is bound via an ester linkage to a carboxyl group present in a block copolymer. When two or more hydroxyl groups of docetaxel are bound to the block copolymer via ester linkages, possible structures may include (i) one docetaxel molecule linked via the ester linkages to two or more carboxyl groups of a single block copolymer or (ii) two or more block copolymers crosslinked via one docetaxel molecule, and the "polymer derivative of docetaxel" includes the both structures.

Examples of the method of linking docetaxel to the block copolymer include, but are not limited to, a method in which the block copolymer is reacted with docetaxel in an organic solvent using a condensation agent such as dicyclohexylcarbodiimide (DCC) and diisopropyl carbodiimide (DIPIC) such that a carboxyl group of polyaspartic acid

is condensed with a hydroxyl group of docetaxel. Specific procedures and conditions for such a method are described, e.g., in Examples 1 and 2 and Comparative Example 1 below.

The term "the ratio of docetaxel molecules linked to the block copolymer" as used herein means the ratio of the number of docetaxel molecules linked to the block copolymer relative to the total number of aspartic acid repeating units per molecule of the block copolymer. The term "the number of docetaxel molecules linked to the block copolymer" as used herein means the number of docetaxel molecules linked to the block copolymer per molecule of the block copolymer.

The present inventors have found that the release rate of docetaxel from a polymer derivative of docetaxel can be controlled by adjusting the ratio and/or the number of docetaxel molecules linked to the block copolymer. Specifically, as the ratio of docetaxel molecules linked to the block copolymer is higher, and as the number of docetaxel molecules linked to the block copolymer is higher, the release rate of docetaxel from the resultant polymer derivative of docetaxel becomes slower, resulting in enhancement of the sustained-release property of the polymer derivative of docetaxel. This finding has enabled preparation of a polymer derivative of docetaxel with reduced side effects and an excellent drug efficacy.

Specifically, the ratio of the number of linked docetaxel molecules relative to the total number of aspartic acid repeating units per molecule of the block copolymer should be adjusted to preferably 28% or higher, more preferably 30% or higher, and most preferably 32% or higher, and the number of linked docetaxel molecules per molecule of the block copolymer should be adjusted to preferably 11 or more, more preferably 12 or more, and most preferably 13 or more. Note that the aforementioned values generally mean averages of two or more polymers.

By adjusting the ratio and/or the number of docetaxel molecules linked to the block copolymer as explained above, it is possible to control the release rate of docetaxel from the polymer derivative of docetaxel in 24 hours to preferably 29% or lower, more preferably 23% or lower, and most preferably 15% or lower.
The term "the release rate" as used herein means the release rate of docetaxel measured at 37°C in a 0.1 M sodium phosphate buffer, pH 7.4.

Examples of the method of adjusting the ratio and/or the number of docetaxel molecules linked to the block copolymer include, but are not limited to, a method including adjusting the ratio of the used amount of the block copolymer to that of docetaxel when linking the block copolymer and docetaxel.

Polymer derivatives of docetaxel producible by the method of the present invention include novel polymer derivatives of docetaxel, which also constitute the present invention.
The polymer derivative of docetaxel of the present invention is a polymer derivative of docetaxel provided by an ester linkage between at least one hydroxyl group of docetaxel and a carboxyl group in an aspartic acid side chain of a block copolymer including polyethylene glycol and polyaspartic acid, in which (i) the ratio of linked docetaxel molecules relative to the total number of aspartic acid repeating units per molecule of the block copolymer is 28% or higher, and/or (ii) the number of linked docetaxel molecules per molecule of the block copolymer is 11 or more.

The polymer derivative of docetaxel of the present invention may preferably be represented by formula (I):

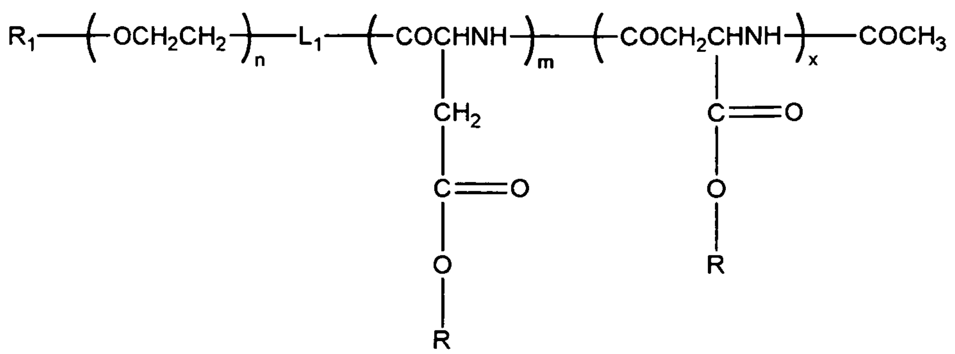

(I)

where $R_1$ is a hydrogen atom or a $C_1$-$C_6$ alkyl group, $L_1$ is a linking group, R is a hydrogen atom or a docetaxel molecule of which at least one hydroxyl group of docetaxel forms the ester linkage, n is an integer of 40 to 450, and m+x is an integer of 35 to 60, provided that 0% to 90% of m+x is x, and that when x is higher than 0, (COCHNH) units and ($COCH_2CHNH$) units are present at random.

$L_1$ linking group in formula (I) may be any group as long as it can link polyethylene glycol to polyaspartic acid, a preferable example of which is $R_5(CH_2)_pR_6$, wherein $R_5$ is O, $R_6$ is NH, and p is an integer of 1 to 6.

In formula (I), n is an integer of 40 to 450, preferably an integer of 60 to 410, and most preferably an integer of 110 to 340, and m+x is an integer of 35 to 60, preferably an integer of 36 to 50. The numerical values of n, m and x are mean values.

The polymer derivative of docetaxel of the present invention may form a polymer micelle with a water-soluble polymer such as polyethylene glycol as the outer shell in the water. Considering its use to humans, the particle diameter of the polymer micelle may preferably be 10 μm or smaller and more preferably 5 μm or smaller. Specifically, it should preferably be 200 nm or less for use in, e.g., intravenous administration.

The polymer derivative of docetaxel of the present invention should preferably be used in pharmaceutical formulations, specifically anti-cancer agents. Examples of formulations containing the polymer derivative of docetaxel of the present invention include, but are not limited to, a solution and a lyophilized formulation, the latter being preferred. In any case, the formulations may contain any additional ingredients commonly used in drug formulation, such as diluents, excipients, isotonic agents and pH-modifying agents.

The polymer derivative of docetaxel of the present invention can be administered by any route but should preferably be administered parenterally, such as subcutaneously, intravenously, intraarterially, or locally, intravenous injection being specifically preferred.

The polymer derivative of docetaxel of the present invention may be administered in any dosage, which can be selected as appropriate, depending on various conditions including the dosage regimen and the age, sex and conditions of the patient. In general, the dosage per day should be, but is not limited to, 1 to 1000 mg/$m^2$, preferably 10 to 700 mg/$m^2$ in terms of docetaxel.

## EXAMPLES

The present invention will now be explained with reference to specific examples, although the present invention is not limited to these examples by any means. Note that docetaxel may also be called "DTX" herein.
Also note that a polymer derivative of docetaxel may be represented by the abbreviation of the polymer with the suffix "-DTX." For example, a polymer derivative in which docetaxel is bound to a polyethylene glycol-polyaspartic acid block copolymer may be represented by "PEG-pAsp-DTX."
In addition, note that when the number n of linked docetaxel molecules per molecule of a polymer derivative is known, the polymer derivative may be represented using the binding number n, as "PEG-pAsp-nDTX" or simply "nDTX." For example, PEG-pAsp-DTX in which the binding number of docetaxel per molecule is 14, it may be represented by "PEG-pAsp-14DTX" or simply "14DTX."

### Example 1

Synthesis of 14DTX

Synthesis of a polymer derivative of docetaxel (PEG-pAsp-14DTX)

560 mg of PEG-pAsp-Ac (the average molecular weight of PEG: 10,000, the average number of aspartic acid residues: 40, the aspartic acid side chain is a carboxyl group) which is a methoxypolyethylene glycol-polyaspartic acid block copolymer (provided that one end of polyaspartic acid has been acetylated) synthesized according to the method described in Patent document 3 was dissolved in 10 mL of dry N,N-dimethylformamide (DMF) (Kanto Kagaku), to which 1.0 g of docetaxel trihydrate (ScinoPharm Taiwan, Ltd.) that had previously been lyophilized was added. Subsequently, 140 mg of 4-dimethylaminopyridine (Wako Pure Chemical Industries, Ltd.) and 177 μl of N,N'-diisopropyl carbodiimide (Kokusan Kagaku) were added sequentially, and the mixture was stirred overnight. The reaction mixture was added dropwise to 500 mL of a mixture of hexane and ethyl acetate (volume ratio 1:1) to crystallize the polymer, which was then suction-filtered. The polymer was suspended in 100 mL of purified water to prepare a polymer micelle. The solution was diluted five-fold in purified water and ultra-filtered (Labscale TFF System manufactured by Millipore, MWCO=100,000). The ultra filtration procedure was repeated five times, and the solution was lyophilized. The polymer obtained was dissolved in 10 mL of dry DMF, and then added dropwise to 500 mL of a mixture of hexane and ethyl acetate (volume ratio 1:1) to crystallize the polymer, which was then suction-filtered. Furthermore, the polymer powder as it was was placed in 500 mL of the same solvent mixture, suction-filtered, and dried under reduced pressure overnight at room temperature to obtain 672 mg of a pale yellow powder of PEG-pAsp-DTX. One mg of it was weighed out, and was dissolved in 10 mL of a mixture solution of purified water and ethanol (volume ratio 1:1). Based on the absorbance of the solution at 233 nm, the amount of bound docetaxel was calculated to be 14 molecules per polymer.

Release property of 14DTX

To 950 μl of a 0.1 M sodium phosphate buffer, pH7.4, that had previously been warmed to 37°C, 50 μl of PEG-pAsp-14DTX (14DTX: the ratio of the number of bound docetaxel molecules relative to the total number of aspartic acid is 35%) micelle obtained as above was added to start a drug release experiment (the final concentration of DTX is 10

μg/ml). Over time, 50 μl of the sample was collected, and the liquid volume was complemented with the same buffer. The concentration of free DTX in the collected sample was determined by high performance liquid chromatography (HPLC). The release rate (%) of the drug was calculated based on the drug content determined from absorbance at 233 nm during micelle preparation. As a result, 3.0% of drug release was observed at 3 hours, 9.0% at 24 hours, and 10.7% at 48 hours after the start of the experiment.

Example 2

Synthesis of 12DTX

One gram of a methoxypolyethylene glycol-polyaspartic acid block copolymer PEG-pAsp-Ac (the aspartic acid side chain is a carboxyl group) that was obtained by treating a methoxypolyethylene glycol-polyaspartic acid benzylester copolymer PEG-PBLA-Ac (provided that one end of polyaspartic acid has been acetylated, the mean molecular weight of PEG: 10,000, the mean number of aspartic acid residues: 40) with a mixture of hydrogen bromide and acetic acid (25% HBr/AcOH: Kokusan Kagaku) and then deprotecting the benzylester was dissolved in 20 mL of dry DMF (Kanto Kagaku), to which 2.2 g of docetaxel anhydrate (ScinoPharm Taiwan, Ltd.) was added. Subsequently, 340 mg of 4-dimethylaminopyridine (Wako Pure Chemical Industries, Ltd.) and 440 μl of N,N'-diisopropyl carbodiimide (Kokusan Kagaku) were added sequentially, and the mixture was stirred overnight. The reaction mixture was added dropwise to 500 mL of a mixture of hexane and ethyl acetate (volume ratio 1:1) to crystallize the polymer, which was then suction-filtered. The polymer was suspended in 300 mL of purified water to prepare a polymer micelle. The solution was made to a volume of 500 mL in purified water and ultra-filtered (Labscale TFF System manufactured by Millipore, MWCO=100,000). The procedure was repeated five times, and the solution was lyophilized. The polymer obtained was dissolved in 20 mL of dry DMF, and then added dropwise to 500 mL of a mixture of hexane and ethyl acetate (volume ratio 1:1) to crystallize the polymer, which was then suction-filtered. The polymer powder as it was was suspended in 500 mL of the same solvent mixture, suction-filtered, dried under reduced pressure overnight at room temperature to obtain 1.22 g of a pale yellow powder of PEG-pAsp-DTX. One mg of it was weighed out, and was dissolved in 10 mL of a mixture of purified water and ethanol (volume ratio 1:1). Based on the absorbance of the solution at 233 nm, the amount of bound docetaxel was calculated to be 12 molecules per polymer.

Release property of 12DTX

To 950 μl of a 0.1 M sodium phosphate buffer, pH7.4, that had previously been warmed to 37°C, 50 μl of PEG-pAsp-12DTX (12DTX: the ratio of the number of bound docetaxel molecules relative to the total number of aspartic acid is 30%) micelle obtained as above was added to start a drug release experiment (the final concentration of DTX is 10 μg/ml). Over time, 50 μl of the sample was collected, and instead the liquid volume was replenished with the same buffer. The concentration of free DTX in the collected sample was determined with HPLC and the release rate (%) of the drug was determined as in Example 1. As a result, 7.8% of drug release was observed at 3 hours, 24.1% at 24 hours, and 31.1% at 48 hours after the start of the experiment.

Comparative Example 1

Synthesis of 5DTX

500 mg of PEG-pAsp-Ac (the mean molecular weight of PEG: 12,000, the mean number of aspartic acid residues: 40, the aspartic acid side chain is a carboxyl group) which is a methoxypolyethylene glycol-polyaspartic acid block copolymer (provided that one end of polyaspartic acid has been acetylated) synthesized according to the method described in Patent document 3 was dissolved in 10 mL of dry DMF (Kanto Kagaku), to which 1.05 g of docetaxel trihydrate (ScinoPharm Taiwan, Ltd.) was added. Subsequently, 150 mg of 4-dimethylaminopyridine (Wako Pure Chemical Industries, Ltd.) and 200 μl of N,N'-diisopropyl carbodiimide (Kokusan Kagaku) were added sequentially, and the mixture was stirred overnight at room temperature. The reaction mixture was added dropwise to 500 mL of a mixture of hexane and ethyl acetate (volume ratio 1:1) to crystallize the polymer, which was then suction-filtered. The polymer was suspended in 100 mL of purified water to prepare a polymer micelle. The solution was diluted five-fold in purified water and ultra-filtered (Labscale TFF System manufactured by Millipore, MWCO=100,000). The ultra filtration procedure was repeated five times, and the solution was lyophilized. The polymer obtained was dissolved in 10 mL of dry DMF, and then added dropwise to 500 mL of a mixture of hexane and ethyl acetate (volume ratio 1:1) to crystallize the polymer, which was suction-filtered. Furthermore, the polymer powder as it was was placed in 500 mL of the same solvent mixture, suction-filtered, dried under reduced pressure overnight at room temperature to obtain 520 mg of a pale yellow powder of PEG-pAsp-DTX. One mg of it was weighed out, and was dissolved in 10 mL of a mixture of purified water and ethanol

(volume ratio 1:1). Based on the absorbance of the solution at 233 nm, the amount of bound docetaxel was calculated to be 5 molecules per polymer.

Release property of 5DTX

To 950 μl of a 0.1 M sodium phosphate buffer, pH7.4, that had previously been warmed to 37°C, 50 μl of PEG-pAsp-5DTX (5DTX: the ratio of the number of bound docetaxel molecules relative to the total number of aspartic acid is 12.5%) micelle obtained as above was added to start a drug release experiment (the final concentration of DTX is 10 μg/ml). Over time, 50 μl of the sample was collected, and the liquid volume was complemented with the same buffer. The concentration of free DTX in the collected sample was determined with HPLC, and the release rate (%) of the drug was calculated as in Example 1. As a result, 21.9% of drug release was observed at 1 hour after the start of the experiment. The drug release rate was 28.6% at 2 hours, 68.0% at 24 hours, and 75.1% at 48 hours.

The time-courses of the drug release rate obtained in Working Examples 1 and 2 and Comparative Example 1 are shown in Fig. 1. Also, a line obtained through plotting the drug release rate at 24 hours relative to the amount of bound docetaxel is shown in Fig. 2.

Example 3

Pharmacokinetic study using PEG-pAsp-14DTX in mice

1) Preparation of a polymer micelle

Ten mg in terms of DTX of the polymer derivative of docetaxel (PEG-pAsp-14DTX) obtained in Example 1 was precisely weighed into a sample vial, to which 1 ml of purified water was added to suspend the sample. After stirring at 4°C for a whole day and night, it was sonicated under ice cooling for 10 minutes using a Biodisruptor (Nihon Seiki Seisakusho, High Power Unit), filtered with a 0.22 μm filter [Millipore, Millex(registered trademark) GP PES], and the filtrate was collected. The filtrate was gel-filtered (GE Healthcare Bioscience, PD-10, the elution solution: a 10% sucrose solution), and the collected polymer micelle fraction (the mean particle size 120 nm) was used in the following experiment.

2) Animal experiment

To male Balb/c mice (Charles River Laboratories Japan, Inc.; 7 week-old), the above DTX polymer micelle (equivalent to docetaxel concentration of 5 mg/mL) was administered from the tail vein under ether anesthesia at a DTX dose of 50 mg/kg (n=3). As the control, the docetaxel solution was similarly administered at 10 mg/kg (n=3). However, the docetaxel solution was prepared by dissolving Taxotere(registered trademark) Injection 20 mg (Sanofi-Aventis K.K.) in a reconstituting solution (13% ethanol) (10 mg/mL) and then diluting 10-fold with a 10% sucrose solution. At each time point, one mouse was sacrificed, and the blood was collected under ether anesthesia at 5 minutes, 1, 6, 24, and 48 hours after the administration. The blood was centrifuged at 4°C, plasma was collected and stored at -30°C until use for measurement.

3) Determination of plasma levels of DTX

a) Determination of total DTX concentration

DTX in the polymer-bound form was hydrolyzed to a free form, and total DTX was determined as follows. To 50 μl of the collected plasma, 200 μl of a 50 mg/mL aqueous solution of $NaHCO_3$, 400 μl of 30% $H_2O_2$, and 300 μl of dichloromethane were added in this order, and stirred for 3 hours at room temperature. Subsequently, after centrifugation (Funakoshi, Chibitan, 10,000 rpm, one minute) at room temperature, the organic phase was collected. To the remaining aqueous phase, 300 μl of dichloromethane was added again, and stirred vigorously for one minute at room temperature. The organic phase was collected by a similar centrifugation, combined with the formerly collected organic phase, and evaporated to dryness under reduced pressure in a centrifuging evaporator. To the dried sample, 100 μL of paclitaxel/50% acetonitrile (20 μg/mL) was added and dissolved. This was loaded in a HPLC sample vial and analyzed by HPLC under the following condition. The content of DTX was calculated based on the ratio of the peak area of DTX relative to the peak area derived from paclitaxel, which was used as the internal standard.

b) Determination of free DTX concentration

To 50 μl of the collected plasma, 20 μl of paclitaxel/50% acetonitrile (20 μg/mL) as the internal standard and

300 μl of ethyl acetate were added in this order, and stirred vigorously for one minute at room temperature. Subsequently, after centrifugation (Funakoshi, Chibitan, 10,000 rpm, one minute) at room temperature, the organic phase was collected. To the remaining aqueous phase, 300 μL of ethyl acetate was added again, and shaken vigorously for one minute at room temperature. The organic phase was collected by a similar centrifugation, combined with the formerly collected organic phase, and evaporated to dryness under reduced pressure in a centrifuging evaporator. To the dried sample, 100 μL of 50% acetonitrile was added and dissolved. This was loaded in a HPLC sample vial and analyzed by HPLC

c) Analytical condition

The HPLC condition is as follows. Unless otherwise specified, HPLC analysis regarding to DTX was carried out under the same condition.

System: Waters Alliance System
Column: Tosoh TSK-gel ODS-80TM (4.6ϕ × 150 mm) (40°C)
Mobile phase: Water/acetonitrile= 55/45
Flow rate: 1 mL/min
Detection: UV (233 nm)
Injection volume: 20 μl

4) Measurement result of changes in plasma levels with time

The result is shown in Fig. 3. In contrast to the docetaxel solution that disappears rapidly, PEG-pAsp-14DTX after administration remained at high levels as total DTX for a long time. It was shown that, in the case of the polymer derivative of docetaxel micelle, docetaxel remains at high levels in the plasma as total DTX for a long time. Also, after the administration of the polymer micelle, free DTX was detected in the plasma. The concentration of free DTX was retained at about 1/100 of the total DTX concentration over the time.

Example 4

(Drug efficacy test using human prostate cancer PC-3 cells)

Using the polymer derivative of docetaxel (PEG-pAsp-14DTX) obtained in Example 1, the following experiment was carried out. PC-3 cells were purchased from ATCC through Summit Pharmaceuticals International Corporation. PC-3 cells were cultured in a RPMI1640 + 10% FBS medium at 5% $CO_2$ and 37°C, and grown to the cell number required for xenograft. The cells were suspended in physiological saline, and inoculated subcutaneously at the back of the male nude mice [Balb nu/nu, 5 week-old, Charles River Laboratories Japan, Inc.] to $3 \times 10^6$ cells/100 μl per mouse. Then, the nude mice were kept for 15 days, and drug administration was started when the tumor volume reached 81.6 ± 5.0 $mm^3$ (mean ± SE, in which SE represents the standard error, the same shall apply hereinafter). The administration schedule was the tail vein administration every four days for a total of three times. For the following four groups (n=8), changes in tumor volume and body weight over time were determined: (1) Control group (untreated), (2) 15 mg/kg of a DMSO solution of docetaxel group, (3) 15 mg/kg of PEG-pAsp-14DTX group, and (4) 20 mg/kg of PEG-pAsp-14DTX group (the dosage was the converted amount of docetaxel mg/kg/injection). In (4) 20 mg/kg of PEG-pAsp-14DTX group, reduction in body weight, which is a side effect, was not observed and thus additional administration was carried out for additional four times. For tumor volume, the major dimension (a mm) and the minor dimension (b mm) were measured with an electronic caliper [Mitsutoyo Corporation], and the volume was calculated according to the following equation.

[Equation I]

$$\text{Tumor volume } (mm^3) = a \times b^2 / 2$$

Changes in tumor volume are shown in Fig. 4 and those in body weight are shown in Fig. 5.
Any of the PEG-pAsp-14DTX 15 mg/kg administration group and the PEG-pAsp-14DTX 20 mg/kg administration group suppressed tumor growth compared to the control group. In the 15 mg/kg of the DMSO solution of docetaxel group as well, tumor growth was markedly suppressed and a marked reduction in body weight was observed.

On the other hand, in the PEG-pAsp-14DTX 20 mg/kg administration group, no reduction in body weight was observed after administration for three times, and thus four additional administrations were carried out, but no reduction in body weight was observed either.
This demonstrated that PEG-pAsp-14DTX has a very low side effect and an excellent therapeutic effect.

Example 5

(Drug efficacy test using human breast cancer MDA-MB-231 cells)

Using the polymer derivative of docetaxel (PEG-pAsp-14DTX) obtained in Example 1, the following experiment was carried out. MDA-MB-231 cells were purchased from ECAC through DS Pharma Biomedical Co., Ltd. MDA-MB-231 cells were cultured in a RPMI1640 + 10% FBS medium at 5% CO2 and 37°C, and grown to the cell number required for xemograft. The cells were suspended in physiological saline, and inoculated subcutaneously at the back of male nude mice [Balb nu/nu, 5 week-old, Charles River Laboratories Japan, Inc.] to $3 \times 10^6$ cells/100 μl per mouse. Then, the mice were kept for 21 days, and drug administration was started when the tumor volume reached $70.8 \pm 3.7$ mm$^3$ (mean ± SE). The administration schedule was the tail vein administration every four days for a total of three times. For the following two groups (n=8), time-coueses of tumor volume and body weight changes were measured 3 times a weak: (1) Control (no treatment), (2) PEG-pAsp-14DTX 10 mg/kg (the dosage was the converted amount of docetaxel mg/kg/injection). Tumor volume was measured as described and calculated as described in Example 2. Changes in tumor volume are shown in Fig. 6, and those in body weight are shown in Fig. 7.

Example 6

(Test on changes in body weight using healthy mice)

To normal male mice (Balb/c mice, 6-7 week-old, Charles River Laboratories Japan, Inc.), three sample of (i) a docetaxel solution, (ii) PEG-pAsp-14DTX (Example 1), and (iii) PEG-pAsp-5DTX (Comparative Example 1) were administered to the tail vein every four days for a total of three times, and temporal changes of their body weight were compared through 4 weeks after the administration. The docetaxel solution was prepared by dissolving Taxotere(registered trademark) Injection 20 mg (Sanofi-Aventis K.K.) in a reconstituting solution (13% ethanol) (10 mg/mL) and then diluting 10-fold in a 10% sucrose solution (1 mg/mL) .
Temporal changes in body weight of each administration group (n=1 to 2) calculated with the body weight at the start of administration being set as 100% are shown in Fig. 8. The three times administration of the docetaxel solution 15 mg/kg cause body weight loss with a maximum ratio of 24.1% on day 14 after the start of administration compared to that at the start of administration. The three times administration of PEG-pAsp-5DTX (Comparative Example 1) 22 mg/kg cause body weight loss with a maximum ratio of 29.8% on day 15 after the start of administration, and exhibited changes relatively similar to the solution administration group. On the other hand, PEG-pAsp-14DTX (Example 1) exhibited changes in body weight not significantly different from those of the untreated group, although the dosage at 30 mg/kg three times is about 1.4 times higher than that of the dosage of Comparative Example 1. These results demonstrated that PEG-pAsp-14DTX causes a marginal body weight loss and can suppress side effects to a minimum.

INDUSTRIAL APPLICABILITY

The present invention can be preferably used in the field of pharmaceutical formulations such as anti-cancer agents in which polymer derivatives of docetaxel are used.

## Claims

1. A polymer derivative of docetaxel provided by an ester linkage between at least one hydroxyl group of docetaxel and a carboxyl group in an aspartic acid side chain of a block copolymer comprising polyethylene glycol and polyaspartic acid, wherein (i) the ratio of the number of linked docetaxel molecules to the total number of aspartic acid repeating units per molecule of the block copolymer is 28% or higher, and/or (ii) the number of linked docetaxel molecules per molecule of the block copolymer is 11 or more

2. The polymer derivative of docetaxel according to claim 1, wherein the release rate of docetaxel in a 0.1 M sodium phosphate buffer, pH 7.4, at 37°C in 24 hours is 29% or lower.

3. The polymer derivative of docetaxel according to claim 1, represented by formula (I)

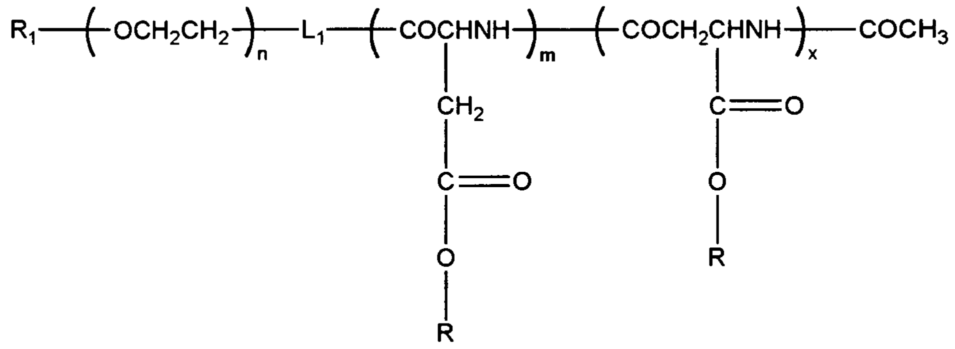

(I)

where $R_1$ is a hydrogen atom or a $C_1$-$C_6$ alkyl group, $L_1$ is a linking group, R is a hydrogen atom or a docetaxel molecule of which at least one hydroxyl group forms the ester linkage, n is an integer of 40 to 450, and m+x is an integer of 35 to 60, provided that 0% to 90% of m+x is x, and that when x is higher than 0, (COCHNH) units and ($COCH_2CHNH$) units are present at random
wherein (i) the ratio of the number of R groups corresponding to docetaxel molecules relative to m+x has been adjusted to 28% or higher, and/or (ii) the number of R groups corresponding to docetaxel molecules has been adjusted to 11 or more.

4. The polymer derivative of docetaxel according to claim 1, represented by formula (I):

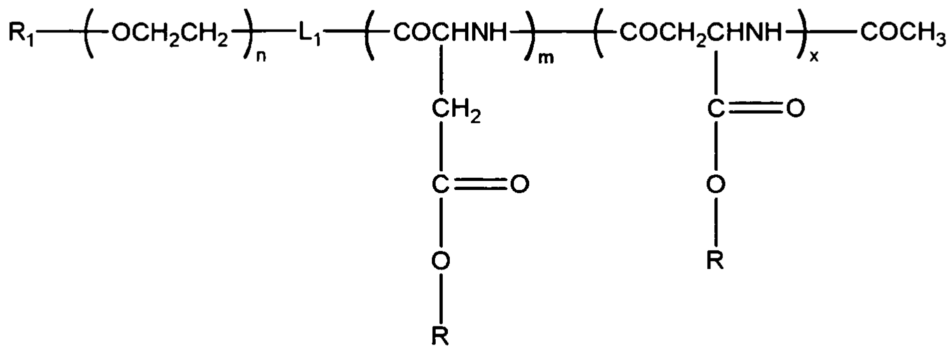

(I)

where $R_1$ is a hydrogen atom or a $C_1$-$C_6$ alkyl group, $L_1$ is a linking group, R is a hydrogen atom or a docetaxel molecule of which at least one hydroxyl group forms the ester linkage, n is an integer of 40 to 450, and m+x is an integer of 35 to 60, provided that 0% to 90% of m+x is x, and that when x is higher than 0, (COCHNH) units and ($COCH_2CHNH$) units are present at random,
wherein (i) the ratio of the number of R groups corresponding to docetaxel molecules relative to m+x has been adjusted to 28% or higher, and/or (ii) the number of R groups corresponding to docetaxel molecules has been adjusted to 11 or more, whereby the release rate of docetaxel in a 0.1 M sodium phosphate buffer, pH 7.4, at 37°C in 24 hours has been inhibited to 29% or lower.

5. The polymer derivative of docetaxel according to claim 1, represented by formula (I):

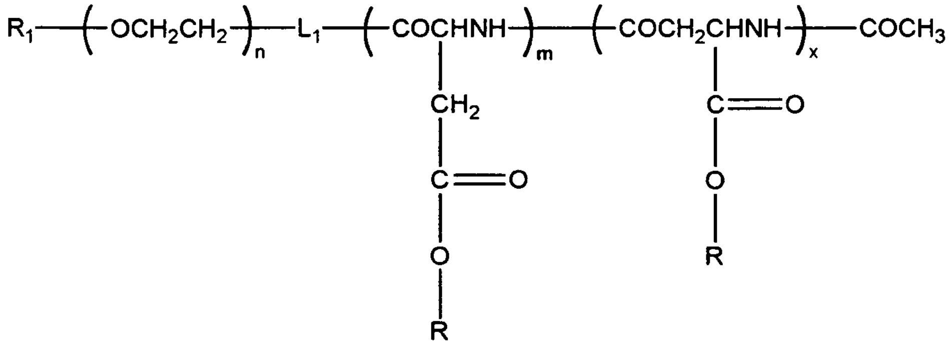

(I)

where $R_1$ is a hydrogen atom or a $C_1$-$C_6$ alkyl group, $L_1$ is a linking group, R is a hydrogen atom or a docetaxel molecule of which at least one hydroxyl group forms the ester linkage, n is an integer of 40 to 450, and m+x is an integer of 35 to 60, provided that 0% to 90% of m+x is x, and that when x is higher than 0, (COCHNH) units and ($COCH_2CHNH$) units are present at random,
wherein (i) the ratio of the number of R groups corresponding to docetaxel molecules relative to m+x has been adjusted to 28% or higher, and/or (ii) the number of R groups corresponding to docetaxel molecules has been adjusted to 11 or more, whereby the release rate of docetaxel in a 0.1 M sodium phosphate buffer, pH 7.4, at 37°C in 24 hours has been inhibited to 29% or lower, and
wherein when the polymer derivative of docetaxel is formulated in the form of a polymer micelle with an outer shell made of a water-soluble polymer having a particle diameter of 200nm or lower and administrated intravenously to an animal, the micelle exhibits anti-tumor effects while preventing loss of the body weight of the animal after the administration.

**6.** An anti-cancer agent comprising the polymer derivative of docetaxel according to any one of claims 1 to 5.

## Patentansprüche

**1.** Docetaxelpolymerderivat bereitgestellt durch eine Esterbindung zwischen mindestens einer Docetaxelhydroxylgruppe und einer Carboxylgruppe in einer Asparaginsäure-Seitenkette eines Blockcopolymers, das Polyethylenglykolund Polyasparaginsäure umfasst, wobei (i) das Verhältnis der Anzahl verbundener Docetaxelmolekülezur Gesamtanzahl der Asparaginsäure-Wiederholungseinheiten pro Molekül des Blockcopolymers28 %oder höherist, und/oder(ii) die Anzahl verbundener Docetaxelmoleküle pro Molekül des Blockcopolymersllodermehr beträgt.

**2.** Docetaxelpolymerderivatnach Anspruch 1,wobei die Freisetzungsrate von Docetaxelin einem 0,1 M Natriumphosphatpuffer, pH 7,4, bei 37°C in24 Stunden 29 %oder niedriger ist.

**3.** Docetaxelpolymerderivatnach Anspruch 1, dargestellt durch Formel (I):

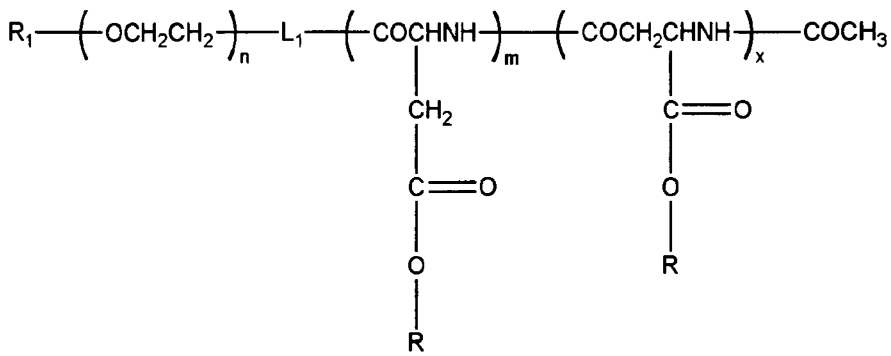

(I)

wobei $R_1$ ein Wasserstoffatom oder eine $C_1$-$C_6$-Alkylgruppe ist, $L_1$ eine Bindungsgruppe ist, R ein Wasserstoffatom

oder ein Docetaxelmolekül ist, von dem mindestens eine Hydroxylgruppe dieEsterbindung bildet, n eine ganze Zahl von 40 bis 450 istund m+x eine ganze Zahl von 35 bis 60ist, vorausgesetzt, dass 0 % bis 90 % von m+xx ist, und dass, wenn x größer als 0 ist, (COCHNH)-Einheiten und ($COCH_2CHNH$)-Einheiten zufällig vorliegen,
wobei (i) das Verhältnis der Anzahl von R-Gruppen, die Docetaxelmolekülen entsprechen,relativ zu m+x auf 28 %oder höher angepasst wurde, und/oder (ii) die Anzahl der R-Gruppen, die Docetaxelmolekülen entsprechen, auf 11 oder mehr angepasst wurde.

**4.** Docetaxelpolymerderivatnach Anspruch 1, dargestellt durch Formel (I):

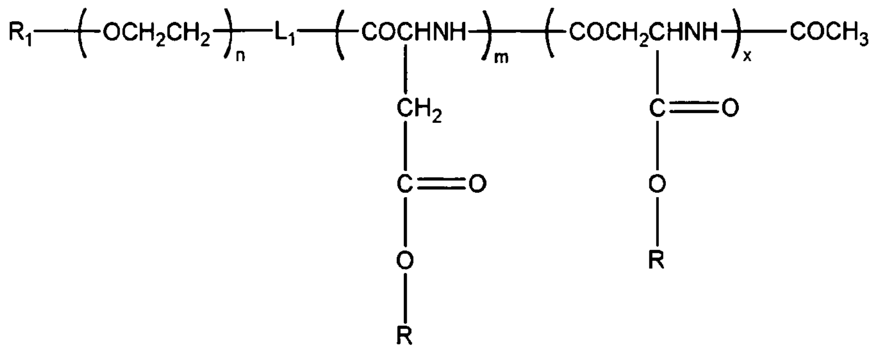

(I)

wobei $R_1$ ein Wasserstoffatom oder eine $C_1$-$C_6$-Alkylgruppe ist, $L_1$ eine Bindungsgruppe ist, R ein Wasserstoffatom oder ein Docetaxelmolekül ist, von dem mindestens eine Hydroxylgruppe dieEsterbindung bildet, n eine ganze Zahl von 40 bis 450 istund m+x eine ganze Zahl von 35 bis 60 ist, vorausgesetzt, dass 0 % bis 90 % von m+xx ist, und dass, wenn x größer als 0 ist, (COCHNH)-Einheiten und ($COCH_2CHNH$)-Einheiten zufällig vorliegen,
wobei (i) das Verhältnis der Anzahl von R-Gruppen, die Docetaxelmolekülen entsprechen,relativ zu m+x auf 28 %oder höher angepasst wurde, und/oder (ii) die Anzahl der R-Gruppen, die Docetaxelmolekülen entsprechen, auf 11 oder mehr angepasst wurde, wodurch die Freisetzungsrate von Docetaxelin einem 0,1 M Natriumphosphatpuffer, pH 7,4,bei 37 °Cin24 Stunden auf 29 %oder niedriger gehemmt wurde.

**5.** Docetaxelpolymerderivatnach Anspruch 1, dargestellt durch Formel (I):

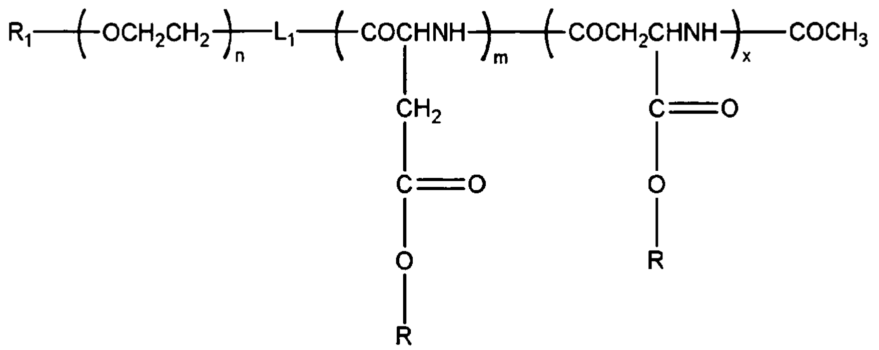

(I)

wobei $R_1$ ein Wasserstoffatom oder eine $C_1$-$C_6$-Alkylgruppe ist, $L_1$ eine Bindungsgruppe ist, R ein Wasserstoffatom oder ein Docetaxelmolekül ist, von dem mindestens eine Hydroxylgruppe dieEsterbindung bildet, n eine ganze Zahl von 40 bis 450 istund m+x eine ganze Zahl von 35 bis 60 ist, vorausgesetzt, dass 0 % bis 90 % von m+xx ist, und dass, wenn x größer als 0 ist, (COCHNH)-Einheiten und ($COCH_2CHNH$)-Einheiten zufällig vorliegen,
wobei (i) das Verhältnis der Anzahl von R-Gruppen, die Docetaxelmolekülen entsprechen,relativ zu m+x auf 28 %oder höher angepasst wurde, und/oder (ii) die Anzahl der R-Gruppen, die Docetaxelmolekülen entsprechen, auf 11 oder mehr angepasst wurde, wodurch die Freisetzungsrate von Docetaxelin einem 0,1 M Natriumphosphatpuffer, pH 7,4,bei 37 °Cin24 Stunden auf 29 %oder niedriger gehemmt wurde, und
wobei, wenn das Docetaxelpolymerderivat in Form einer Polymermizelle mit einer Außenhülle aus einem wasserlöslichen Polymer mit einem Partikeldurchmesser von 200 nm oder weniger formuliert ist und intravenös an ein Tier

verabreicht wird, die Mizelle Antitumorwirkungen exhibiert, während der Körpergewichtsverlust des Tieres nach der Verabreichung verhindert wird.

**6.** Antikrebsmittel, welches das Docetaxelpolymerderivatnach eines der Ansprüche 1 bis 5 umfasst.

**Revendications**

**1.** Dérivé de polymère de docétaxel fourni par une liaison ester entre au moins un groupement hydroxyle de docétaxel et un groupement carboxyle dans une chaîne latérale d'acide aspartique d'un copolymère séquencé comprenant du polyéthylène-glycol et de l'acide aspartique, dans lequel (i) le rapport du nombre de molécules de docétaxel liées au nombre total d'unités récurrentes d'acide aspartique par molécule du copolymère séquencé est de 28 % ou plus et/ou (ii) le nombre de molécules de docétaxel liées par molécule du copolymère séquencé est de 11 ou plus.

**2.** Dérivé de polymère de docétaxel selon la revendication 1, dans lequel le taux de libération de docétaxel dans un tampon de phosphate de sodium à 0,1 M, pH 7,4, à 37 °C en 24 heures, est de 29 % ou moins.

**3.** Dérivé de polymère de docétaxel selon la revendication 1, représenté par la formule (I) :

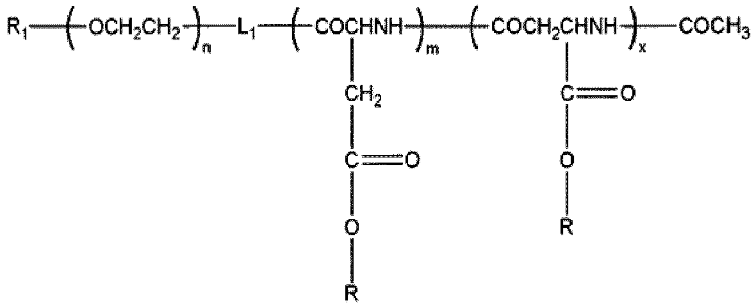

(I)

dans laquelle $R_1$ est un atome d'hydrogène ou un groupement alkyle en $C_1$-$C_6$, $L_1$ est un groupement de liaison, R est un atome d'hydrogène ou une molécule de docétaxel dont un groupement hydroxyle forme la liaison ester, n est un entier de 40 à 450 et m+x est un entier de 35 à 60, pourvu que x constitue 0 à 90 % de m+x et que, lorsque x est supérieur à 0, des unités (COCHNH) et des unités ($COCH_2CHNH$) soient présentes de manière aléatoire, dans lequel dérivé, (i) le rapport du nombre de groupements R correspondant aux molécules de docétaxel par rapport à m+x a été ajusté à 28 % ou plus et/ou (ii) le nombre de groupements R correspondant aux molécules de docétaxel a été ajusté à 11 ou plus.

**4.** Dérivé de polymère de docétaxel selon la revendication 1, représenté par la formule (I) :

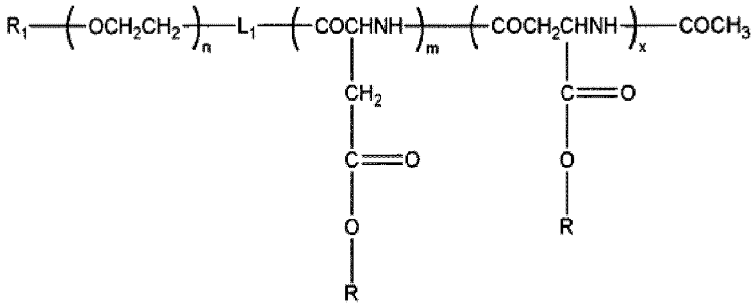

(I)

dans laquelle $R_1$ est un atome d'hydrogène ou un groupement alkyle en $C_1$-$C_6$, $L_1$ est un groupement de liaison, R est un atome d'hydrogène ou une molécule de docétaxel dont un groupement hydroxyle forme la liaison ester, n est un entier de 40 à 450 et m+x est un entier de 35 à 60, pourvu que x constitue 0 à 90 % de m+x et que, lorsque x est supérieur à 0, des unités (COCHNH) et des unités ($COCH_2CHNH$) soient présentes de manière aléatoire, dans lequel dérivé, (i) le rapport du nombre de groupements R correspondant aux molécules de docétaxel par

rapport à m+x a été ajusté à 28 % ou plus et/ou (ii) le nombre de groupements R correspondant aux molécules de docétaxel a été ajusté à 11 ou plus, le taux de libération de docétaxel dans un tampon de phosphate de sodium à 0,1 M, pH 7,4, à 37 °C en 24 heures, ayant été inhibé à 29 % ou moins.

**5.** Dérivé de polymère de docétaxel selon la revendication 1, représenté par la formule (I) :

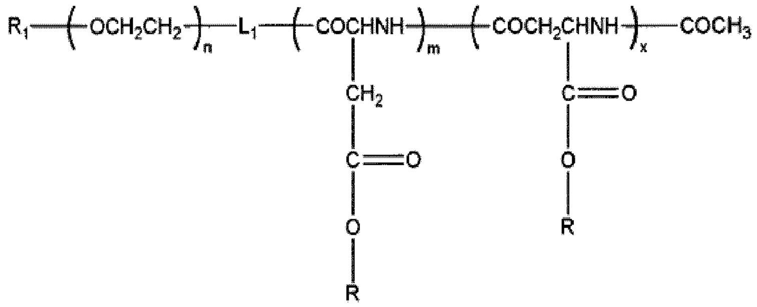

(I)

dans laquelle $R_1$ est un atome d'hydrogène ou un groupement alkyle en $C_1$-$C_6$, $L_1$ est un groupement de liaison, R est un atome d'hydrogène ou une molécule de docétaxel dont un groupement hydroxyle forme la liaison ester, n est un entier de 40 à 450 et m+x est un entier de 35 à 60, pourvu que x constitue 0 à 90 % de m+x et que, lorsque x est supérieur à 0, des unités (COCHNH) et des unités ($COCH_2CHNH$) soient présentes de manière aléatoire, da ns lequel dérivé, (i) le rapport du nombre de groupements R correspondant aux molécules de docétaxel par rapport à m+x a été ajusté à 28 % ou plus et/ou (ii) le nombre de groupements R correspondant aux molécules de docétaxel a été ajusté à 11 ou plus, le taux de libération de docétaxel dans un tampon de phosphate de sodium à 0,1 M, pH 7,4, à 37 °C en 24 heures, ayant été inhibé à 29 % ou moins, et
dans lequel, lorsque le dérivé de polymère de docétaxel se présente sous la forme d'une micelle de polymère avec une enveloppe externe formée d'un polymère soluble dans l'eau ayant un diamètre particulaire de 200 nm ou moins et administrée par voie intraveineuse à un animal, la micelle présente des effets anti-tumoraux tout en empêchant une perte de poids corporel de l'animal après l'administration.

**6.** Agent anti-cancer comprenant le dérivé de polymère de docétaxel selon l'une quelconque des revendications 1 à 5.

Fig.1

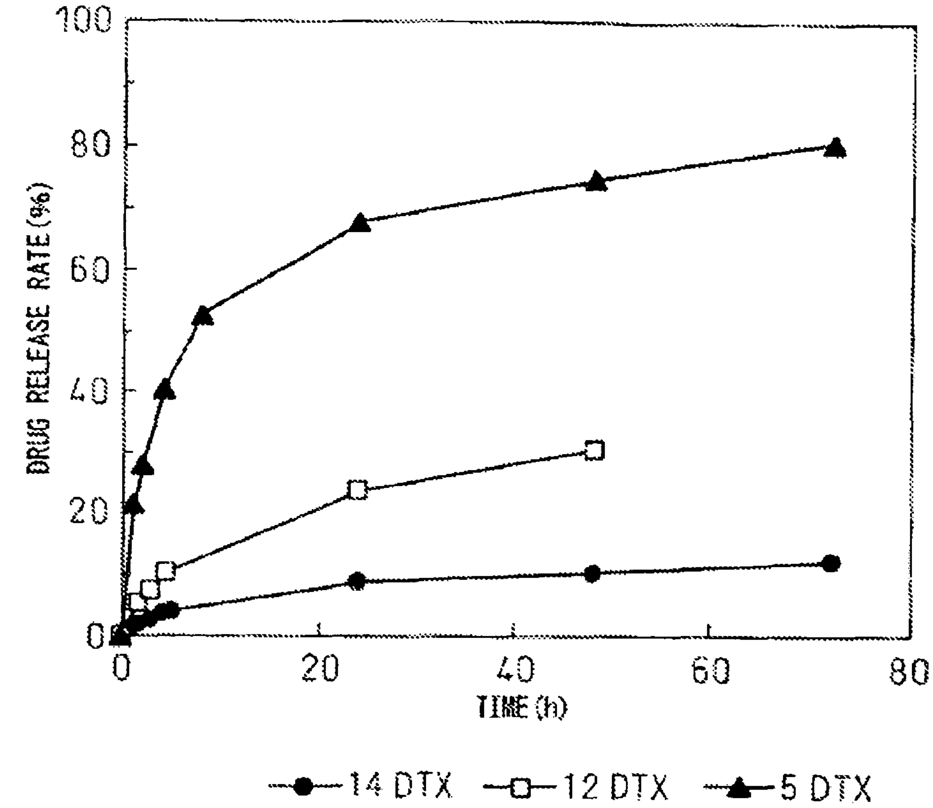
100
80
60
40
20
0
DRUG RELEASE RATE (%)
0
20
40
60
80
TIME (h)
14 DTX
12 DTX
5 DTX

Fig.2

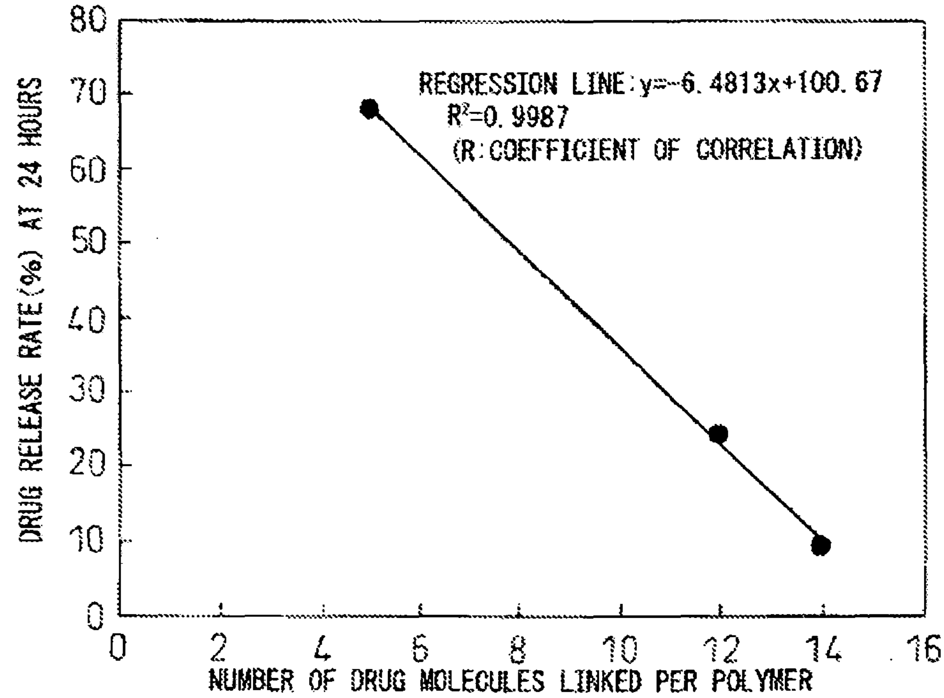
80
70
60
50
40
30
20
10
0
DRUG RELEASE RATE (%) AT 24 HOURS
REGRESSION LINE: y=-6.4813x+100.67
R²=0.9987
(R: COEFFICIENT OF CORRELATION)
0
2
4
6
8
10
12
14
16
NUMBER OF DRUG MOLECULES LINKED PER POLYMER

Fig.3

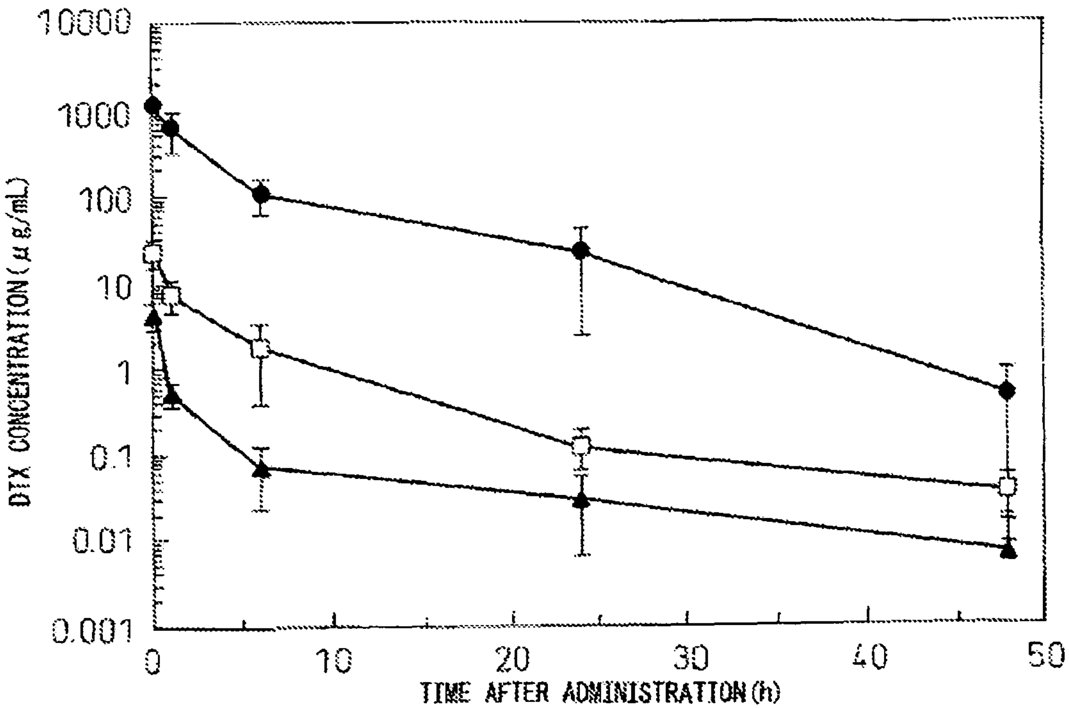
10000
1000
100
10
1
0.1
0.01
0.001
0
10
20
30
40
50
DTX CONCENTRATION (μg/mL)
TIME AFTER ADMINISTRATION (h)

Fig.4

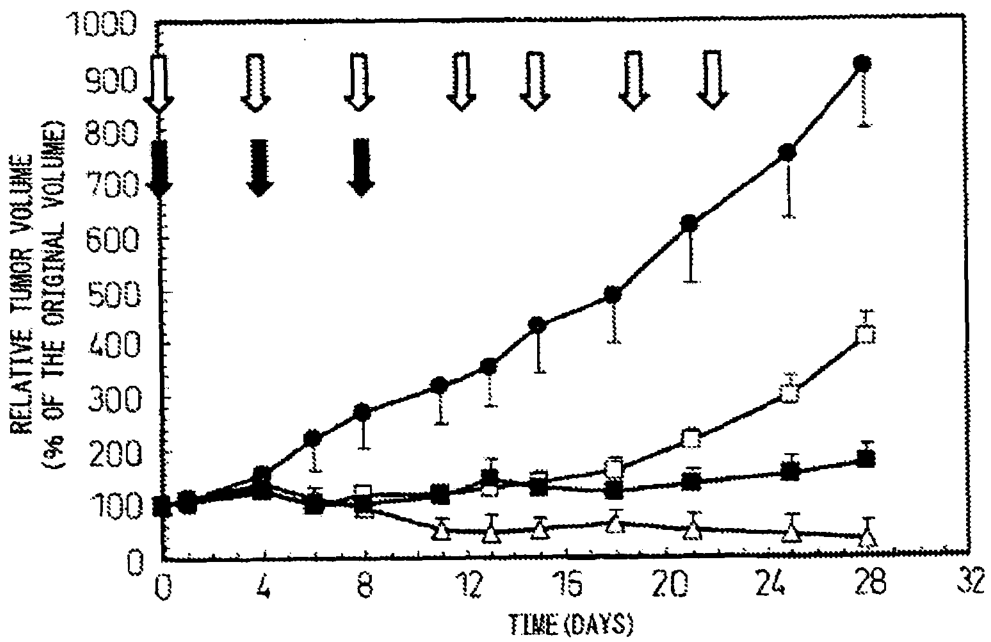
1000
900
800
700
600
500
400
300
200
100
0
0
4
8
12
16
20
24
28
32
RELATIVE TUMOR VOLUME
(% OF THE ORIGINAL VOLUME)
TIME (DAYS)

Fig.5

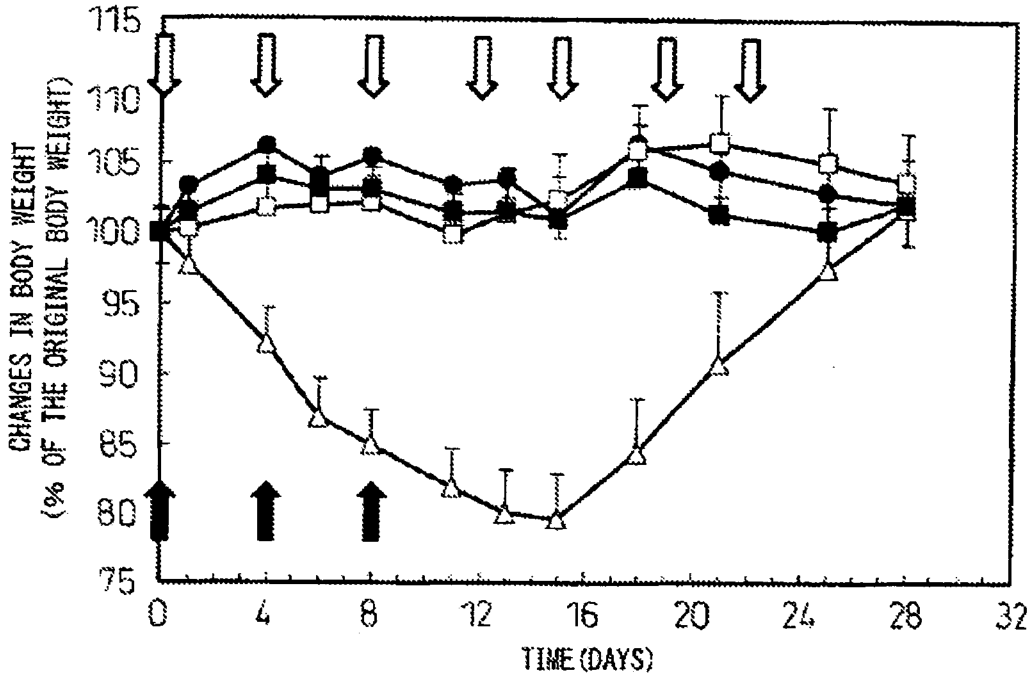
CHANGES IN BODY WEIGHT
(% OF THE ORIGINAL BODY WEIGHT)
115
110
105
100
95
90
85
80
75
0
4
8
12
16
20
24
28
32
TIME (DAYS)

Fig.6

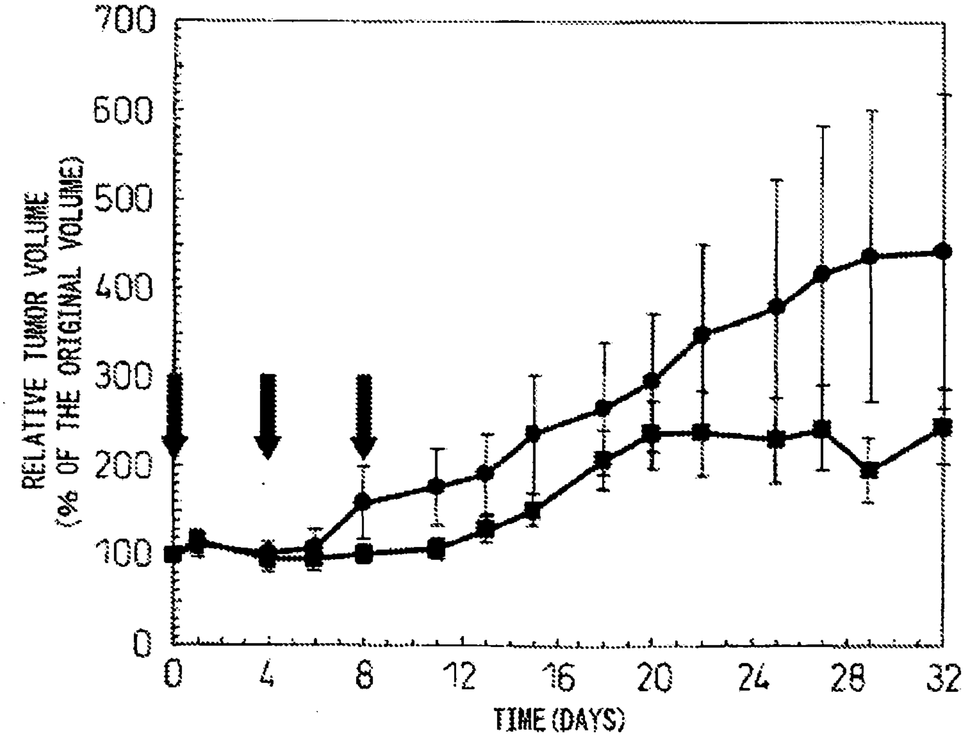
RELATIVE TUMOR VOLUME
(% OF THE ORIGINAL VOLUME)
700
600
500
400
300
200
100
0
0
4
8
12
16
20
24
28
32
TIME (DAYS)

Fig.7

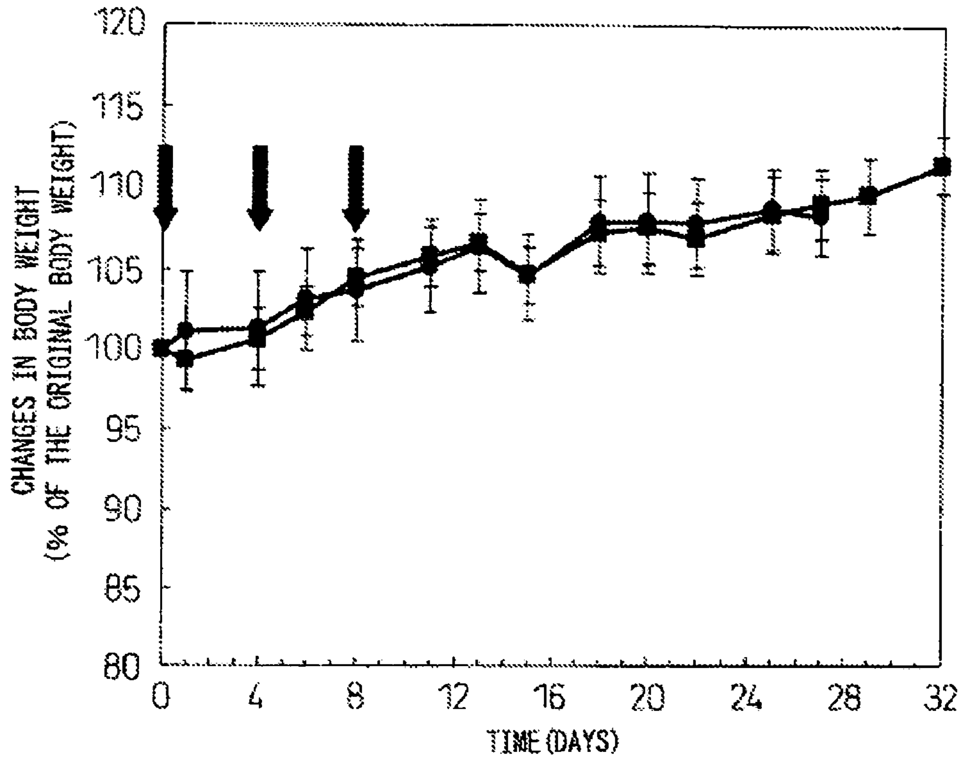
CHANGES IN BODY WEIGHT
(% OF THE ORIGINAL BODY WEIGHT)
120
115
110
105
100
95
90
85
80
0
4
8
12
16
20
24
28
32
TIME (DAYS)

Fig.8

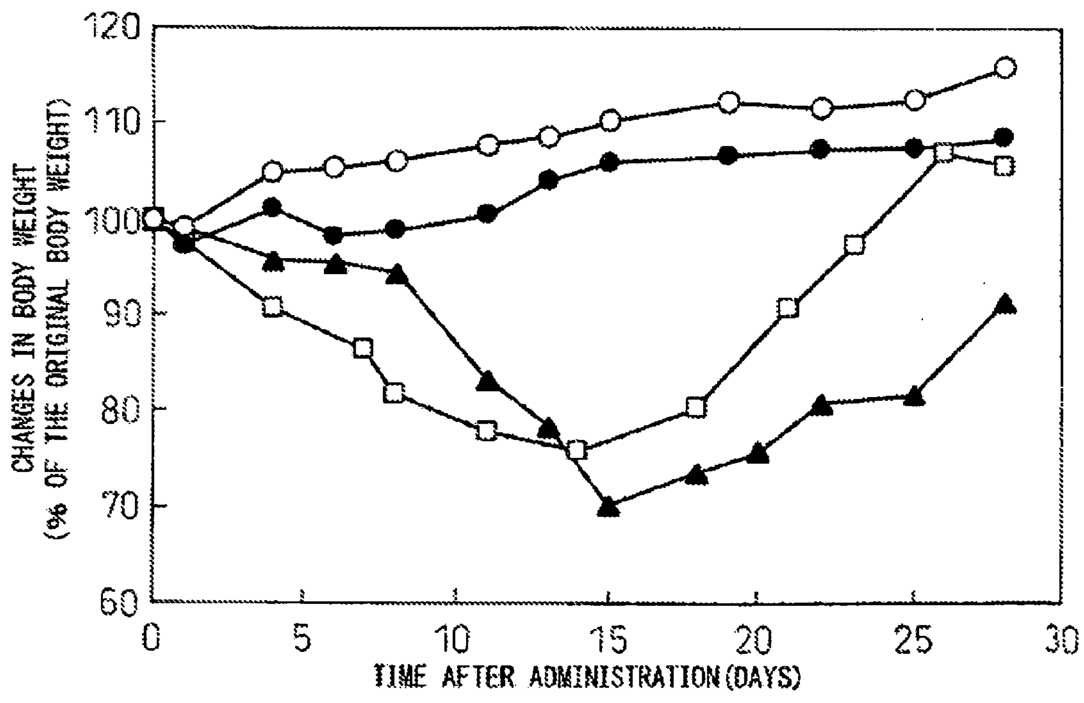
CHANGES IN BODY WEIGHT
(% OF THE ORIGINAL BODY WEIGHT)
120
110
100
90
80
70
60
0
5
10
15
20
25
30
TIME AFTER ADMINISTRATION (DAYS)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1127570 A
- WO 2004082718 A
- JP 2300133 A
- WO 2004039869 A
- WO 2007111211 A